# EUROPEAN PATENT APPLICATION

(11) **EP 1 026 242 A1**
(43) Date of publication of application: **09.08.2000**
(21) Application number: 98950331.3
(22) Date of filing: 21.10.1998
(51) Int. Cl.: C12N 15/10

(54) **METHOD FOR SCREENING FULL-LENGTH cDNA CLONES**

(30) Priority: 22.10.1997 JP 28998297
(71) Applicant: Helix Research Institute, Chiba 292-0812 (JP)
(72) Inventor: OTA, Toshio, Kisarazu-shi Chiba 292-0801 (JP); NISHIKAWA, Tetsuo, Kisarazu-shi Chiba 292-0833 (JP); SALAMOV, Asaf, Saffron Walden Essex CB10 2AP (GB); ISOGAI, Takao, Kisarazu-shi Chiba 292-0833 (JP)
(74) Representative: VOSSIUS & PARTNER
(86) International application number: JP9804772
(87) International publication number: WO9920750

(57) **Abstract**

A method for efficiently screening full-length cDNA clones which comprises: determining the base sequence in the 5'-region of each clone contained in a cDNA library prepared by a method for constructing a cDNA library involving full-length ones at a high ratio; examining the presence/absence of initiation ATG in this 5'-region and the location thereof by using an originally developed software for anticipating initiation codons in cDNA; thus exactly judging the presence/absence of the initiation codon and the location thereof; and screening the cDNAs thus judged as carrying the initiation codon from the cDNA library. Moreover, a cDNA library containing full-length ones at an extremely high ratio can be constructed by mixing the clones thus selected above.

SEQUENCE LISTING

<110> Helix Research Institute, Inc.

<120> Method for screening full-length cDNA clones

<130> H1-806PCT

<150> JP 09-289982
<151> 1997-10-22

<160> 18

<170> PatentIn version 2.0

<210> 1
<211> 30
<212> DNA
<213> Artificial Sequence

<220>
<223> Oligo-capping linker sequence

<400> 1
AGCAUCGAGU CGGCCUUGUU GGCCUACUGG 30

<210> 2
<211> 42
<212> DNA
<213> Artificial Sequence

<220>
<223> Oligo(dT) adapter primer sequence

<400> 2
GCGGCTGAAG ACGGCCTATG TGGCCTTTTT TTTTTTTTTT TT 42

<210> 3
<211> 32
<212> DNA
<213> Artificial Sequence

<220>
<223> Random adapter primer sequence

<400> 3
GCGGCTGAAG ACGGCCTATG TGGCCNNNNN NC 32

<210> 4

<211> 880
<212> DNA
<213> Homo sapiens

<400> 4
ATGCGCCCGC GCGGCCCTAT AGGCGCCTCC TCCGCCCGCC GCCCGGGAGC CGCAGCCGCC 60
GCCGCCACTG CCACTCCCGC TCTCTCAGCG CCGCCGTCGC CACCGCCACC GCCACTGCCA 120
CTACCACCGT CTGAGTCTGC AGTCCCGAGA TCCCAGCCAT CATGTCCATA GAGAAGATCT 180
GGGCCCGGGA GATCCTGGAC TCCCGCGGGA ACCCCACAGT GGAGGTGGAT CTCTATACTG 240
CCAAAGGTCC TTTCCGGGCT GCAGTGCCCA GTGGAGCCTC TACGGGCATC TATGAGGCCC 300
TGGAGCTGAG GGATGGAGAC AAACAGCGTT ACTTAGGCAA AGGTGTCCTG AAGGCAGTGG 360
ACCACATCAA CTCCACCATC GCGCCAGCCC TCATCAGCTC AGGTCTCTCT GTGGTGGAGC 420
AAGAGAAACT GGACAACCTG ATGCTGGAGT TGGATGGGAC TGAGAACAAA TCCAAGTTTG 480
GGGCCAATCC ATCCTGGGTG TGTCTCTGGC CGTGTGTAAG GCANGGGCAA CTGAACNGGA 540
ACTGCCCCTG TATCGCCACA TTGCTCAGCT TGGNCGGGAA CTCANACCTC ATCCTGCCTG 600
TTGCCGGCCT TCAACGTGAT CAATGGTTGG CTTCTCATGC CTGGCAACAA ANCTGGCCAT 660
TGCNGGAATT TTCATGATCC TCCCCNTTGG GAAACTGAAA AACTTTCCGG AATGCCCNTC 720
CAACTAAGTT GCAAAAGGTC TACCNATACC CCCCAAGGGG AATTCCTCCA AGGGAACAAA 780
TNCCCGGGAA AGGAATGCCC CCCAATTNTT NGGGGGAATA AAAGGTGGGC TTTGCCCCCC 840
CATTTTCCTG GAAAAAACNA TNAAAACCCT TGGGAAACTT 880

<210> 5

<211> 645
<212> DNA
<213> Homo sapiens

<400> 5
TGTGCGTTAC TTACCTCNAC TCTTAGCTTG TCGGGGACGG TAACCGGGAC CCGGTGTCTG 60
CTCCTGTCGC CTTCGCCTCC TAATCCCTAG CCACTATGCG TGAGTGCATC TCCATCCACG 120
TTGGCCAGGC TGGTGTCCAN ATTGGCAATG CCTGCTGGGA GCTCTACTGC CTGGAACACG 180
GCATCCAGCC CGATGGCCAG ATGCCAAGTG ACAAGACCAT TGGGGGAGGA GATGACTCCT 240
TCAACACCTT CTTCAGTGAG ACGGGCGCTG GCAANCACGT GCCCCGGGCT GTGTTTGTAG 300
ACTTGGAACC CACAGTCATT GATGAAGTTC GCACTGGCAC CTACCGCCAG CTCTTCCACC 360
CTGAGCAGCT CATCNCAGGC AAGGAAGATG CTGCCAATAA CTATGCCCGA GGGCACTACA 420
CCATTGGCAA GGAGATCATT GACCTTGTGT TGGACCGAAT TCGCAAGCTG GCTGACCANT 480
GCACCGGTCT TCANGGCTTC TTGGTTTTCC ACAGCTTTGG TGGGGGAACT GGTTCTGGGT 540
TCACCTCCCT GCTCATGGAA CGTCTCTCAG TTGATTATGG CAAGAAATCC AAGCTGGAGT 600
TCTCCATTTA CCCAGCACCC CNGGTTTCCN CNGCTGTANT TNGAA 645

<210> 6

<211> 820
<212> DNA
<213> Homo sapiens

<400> 6
CTTTTTTCGC AACGGGTTTG CCGCCAGAAC ACAGGTGTCG TGAAAACTAC CCCTAAAAGC 60
CAAAATGGGA AAGGAAAAGA CTCATATCAA CATTGTCGTC ATTGGACACG TAGATTCGGG 120
CAAGTCCACC ACTACTGGCC ATCTGATCTA TAAATGCGGT GGCATCGACA AAAGAACCAT 180
TGAAAAATTT GAGAAGGAGG CTGCTGAGAT GGGAAAGGGC TCCTTCAAGT ATGCCTGGGT 240
CTTGGATAAA CTGAAAGCTG AGCGTGAACG TGGTATCACC ATTGATATCT CCTTGTGGAA 300
ATTTGAGACC AGCAAGTACT ATGTGACTAT CATTGATGCC CCAGGACACA GAGACTTTAT 360
CAAAAACATG ATTACAGGGA CATCTCAGGC TGACTGTGCT GTCCTGATTG TTGCTGCTGG 420
TGTTGGTGAA TTTGAAGCTG GTATCTCCAA GAATGGGCAG ACCCGAGAGC ATGCCCTTCT 480
GGCTTACACA CTGGGTGTGA AACAACTAAT TGTCGGTGTT AACAAAATGG ATTCACTGAN 540
CCACCCTACA GCCAGAAGAA ATATGANGAA ATTGTTAAGG AAGTCAGCAC TTACATTAAG 600
AAAATTGGCT ACAACCCCGA CACAGTANCA TTTGTGCCAA TTTCTGGTTG GAATGGTGAC 660

AACATGCTGG AACCAANTGC TAACATGCCT TGGTTCCAGG GATGGAAAAT CCCCCNTTAA 720
GGATGGCNAT GCCATTGGAA CCCCCCTGCT TGAAGGCTCT GGANTGCATC CTANCACCAA 780
CTCCTTCAAA TTGAAAAACC CCTTGCNCCC GCCTCCNCCA 840

<210> 7

<211> 788
<212> DNA
<213> Homo sapiens

<400> 7
GAGGCTGAGG CAGTGGCTCC TTGCACAGCA GCTGCACGCG CCGTGGCTCC GGATCTCTTC 60
GTCTTTGCAG CGTAGCCCGA GTCGGTCAGC GCCGGAGGAC CTCAGCAGCC ATGTCGAAGC 120
CCCATAGTGA AGCCGGGACT GCCTTCATTC AGACCCAGCA GCTGCACGCA GCCATGGCTG 180
ACACATTCCT GGAGCACATG TGCCGCCTGG ACATTGATTC ACCACCCATC ACAGCCCGGA 240
ACACTGGCAT CATCTGTACC ATTGGCCCAG CTTCCCGATC AGTGGAGACG TTGAAGGAGA 300
TGATTAAGTC TGGAATGAAT GTGGCTCGTC TGAACTTCTC TCATGGAACT CATGAGTACC 360
ATGCGGAGAC CATCAAGAAT GTGCGCACAG CCACGGAAAG CTTTGCTTCT GACCCCATCC 420
TCTACCGGCC CGTTGCTGTG GCTCTAGACA CTAAAGGACC TGAGATCCGA ACTGGGCTCA 480
TCAAGGGCAG CGGCACTGCA GAGGTGGAGC TGAAGAATGG AGCCACTCTC AAAATCACGC 540
TGGATAATGC CTACATGGAA AAGTGTGACG AGAACATCCT GTGGCTGGAC TACAAGAACA 600
TCTGCAAGGT GGTGGAAGTG GGCAACAAGA TCTACGTGGA TGATGGGCTN ATTTCTCTCC 660
AGGTGAACAC AAAGGTGCCG ACTTCCTGGG TGACNGANGT GGAAAATGGT GGCTCCTTGG 720
GCNCAAGAAA GGTGTGAACT TCCTGGGGCT GCTGTGGANT TGCCTGCTGT GTCNGAAAAA 780
GACATCCA 788

<210> 8

<211> 608
<212> DNA
<213> Homo sapiens

<400> 8
ACAGCCTGGC TCCTTTGAGT ATGAATATGC CATGCGCTGG AAGGCACTCA TTGAGATGGA 60
GAAGCAGCAG CAGGACCAAG TGGACCGCAA CATCNAGGAG GCTCGTGAGA AGCTGGAGAT 120
GGAGATGGAA GCTGCACGCC ATGAGCACCA GGTCATGCTA ATGAGACAGG ATTTGATGAG 180

GCGCCAAGAA GAACTTCGGA GGATGGAAGA GCTGCACAAC CAAGANGTGC AAAAACGAAA 240
GCAACTGGAG CTCAGGCAGG AGGAANAGCG CAGGCGCCGT GAAGAANAGA TGCGGCGGCA 300
GCAAGAAGAA ATGATGCGGC GACNGCAGGA AGGATTCAAG GGAACCTTCC CTGATGCGAG 360
AGAGCAGGAG ATTCGGATGG GTCNGATGGC TATGGGAGGT GCTATGGGCA TAAACNACAG 420
ATGTGCCATG CCCCCTGCTC CTGTGCCAGC TGGTACCCCA GCTCCTCCAG GACCTGCCAC 480
TATTATGCCG GATGGAACTT TGGGATTGAC CCCACCNACA ACTGAACGCT TTGGTCNGGC 540
TGCTACNATG GAANGAATTG GGGCAATTGG TGGAACTCCT CCTGCATTCN ACCGTGCAGC 600
TCCTGGGA 608

<210> 9

<211> 869
<212> DNA
<213> Homo sapiens

<400> 9
ATATTAAACT AGTGAAGCAA CTAAGAGAAA ATGTTAAGTC TGCTATTGAT CTTGAAGAGA 60
TGGCATCTGG TCTTAACAAA AGAAAAATGA TTCAGCATGC TGTATTTAAA GAACTTGTGA 120
AGCTTGTAGA CCCTGGAGTT AAGGCATGGA CACCCACTAA AGGAAAACAA AATGTGATTA 180
TGTTTGTTGG ATTGCAAGGG AGTGGTAAAA CAACAACATG TTCAAAGCTA GCATATTATT 240
ACCAGAGGAA AGGTTGGAAG ACCTGTTTAA TATGTGCAGA CACATTCAGA GCAGGGGCTT 300
TTGACCAACT AAAACAGAAT GCTACCAAAG CAAGAATTCC ATTTTATGGA AGCTATACAG 360
AAATGGATCC TGTCATCATT GCTTCTGAAG GAGTAGAGAA ATTTAAAAAT GAAAATTTTG 420
AAATTATTAT TGTTGATACA AGTGGCCGCC ACAAACAAGA AGACTCTTTG TTTGAAGAAA 480
TGCTTCAAGT TGCTAATGCT ATACAACCTG ATAACATTGT TTATGTGATG GATGCCTCCA 540
TTGGGCAGGC TTGTGAAGCC CAGGCTAAGG CTTTTAAAGA TAAAGTAGAT GTACCTCAGT 600
AATAGTGACA AAACTTGATG GCCATGCAAA ANGAAGTGGT GCACTCAGTG CAGTCGCTGC 660
CACAAAAAAT CCGATTATTT TCATTGGTAC AGGGGGAACA TATANATGAC TTTGAACCTT 720
TCAAAAACAC AGCCTTTTAT TAACAAACTT CTTGGTATNG GCGACATTGA AAGGACTGAT 780
AAATAAAGTC CACNAATTGA AATTTGGATG ACNATGNAAA CCCTTATTGA AAAAATTGAA 840
ACATNGTCCA GTTTTACTTT GCGAAACNT 869

<210> 10

<211> 813
<212> DNA

<213> Homo sapiens

<400> 10
GTTGTGGTAT CTGTATTAAG AAATGCCCCT TTGGCGCCTT ATCAATTGTC AATCTACCAA 60
GCAACTTGGA AAAAGAAACC ACACATCGAT ATTGTGCCAA TGCCTTCAAA CTTCACAGGT 120
TGCCTATCCC TCGTCCAGGT GAAGTTTTGG GATTAGTTGG AACTAATGGT ATTGGAAAGT 180
CAACTGCTTT AAAAATTTTA GCAGGAAAAC AAAAGCCAAA CCTTGGAAAG TACGATGATC 240
CTCCTGACTG GCAGGAGATT TTGACTTATT TCCGTGGATC TGAATTACAA AATTACTTTA 300
CAAAGATTCT AGAAGATGAC CTAAAAGCCA TCATCAAACC TCAATATGTA GACCAGATTC 360
CTAAGGCTGC AAAGGGGACA GTGGGATCTA TTTTGGACCG AAAAGATGAA ACAAAGACAC 420
AGGCAATTGT ATGTCAGCAG CTTGATTTAA CCCACCTAAA AGAACGAAAT GTTGAAGATC 480
TTTCAGGAGG AGAGTTGCAG AGATTTGCTT GTGCTGTCGT TTGCATACAG AAAGCTGATA 540
TTTTCATGTT TGATGAGCCT TCTAGTTACC TAGATGTCAA GCAGCGTTTA AAGGCTGCTA 600
TTACTATACG ATCTCTAATA AATCCAGATA GATATATCAT TGTGGTGGAA CATGATCTAA 660
GTGTATTAGA CTATCTCTCC GACTTCATCT GCTGTTTATA TGGTGTACCA AGCGCCTATG 720
GAATTGTCAC TATGCCTTTT AGTGTTAGAA AAGGCATAAA CNTTTTTTGG ATGGGTATGT 780
TCCAACAGAA AACTTGANAA TCNNAAATGC NTC 813

<210> 11

<211> 655
<212> DNA
<213> Homo sapiens

<400> 11
AGACTCTCAC CGCAGCGGCC AGGAACGCCA GCCGTTCACG CGTTCGGTCC TCCTTGGCTG 60
ACTCACCGCC CTCGCCGCCG CACCATGGAC GCCCCCAGGC AGGTGGTCAA CTTTGGGCCT 120
GGTCCCGCCA AGCTGCCGCA CTCAGTGTTG TTAGAGATAC AAAAGGAATT ATTAGACTAC 180
AAAGGAGTTG GCATTAGTGT TCTTGAAATG AGTCACAGGT CATCAGATTT TGCCAAGATT 240
ATTAACAATA CAGAGAATCT TGTGCGGGAA TTGCTAGCTG TTCCAGACAA CTATAAGGTG 300
ATTTTTCTGC AAGGAGGTGG GTGCGGCCAG TTCAGTGCTG TCCCCTTAAA CCTCATTGGC 360
TTGAAAGCAG GAAGGTGTGC GGACTATGTG GTGACAGGAG CTTGGTCAGC TAAGGCCGCA 420
GAAGAAGCCA AGAAGTTTGG GACTATAAAT ATCGTTCACC CTAAACTTGG GAGTTATACA 480
AAAATTCCAG ATCCAAGCAC CTGGAACCTC AACCCANATG CCTCCTACGT GTTTTATTGC 540
NCAAATGAAA CGGTGCATGG TGTTGANTTT GACTTTATAC CCNATGTCAA GGGAACANTA 600
CTGGTTTGTG ACATTTTCCT CCAACTTCCT GTCCAANCCA ATTGNATGTT TCCAA 655

<210> 12

<211> 599
<212> DNA
<213> Homo sapiens

<400> 12
AAAGATGCGC AGGCGCCGTG TGGCACTCGG CGGTCGAAAG GGGAGTTCAA GGAGACGGGG 60
GCGACGCGGC TGAGGGCTTC TCGTCGGGGT CGGGGCTGCA GCCGTCATGC CGGGGATAGT 120
GGAGCTGCCC ACTCTAGAGG AGCTGAAAGT AGATGAGGTG AAAATTAGTT CTGCTGTGCT 180
TAAAGCTGCG GCCCATCACT ATGGAGCTCA ATGTGATAAG CCCAACAAGG AATTTATGCT 240
CTGCCGCTGG GAANAGAAAG ATCCGAGGCG GTGCTTAGAG GAAGGCAAAC TGGTCAACAA 300
GTGTGCTTTG GACTTCTTTA GGCAGATAAA ACGTCACTGT GCAGAGCCTT TTACAGAATA 360
TTGGACTTGC ATTGATTATA CTGGCCAGCA GTTATTTCGT CACTGTCGCA AACAGCAGGC 420
AAAGTTTGAC NAGTGTGTGC TGGACAAACT GGGCTGGGTG CGGCCTGACC TGGGAAAACT 480
GTCAAAGGTC ACCAAAGTGA AAACAGATCN ACCTTTACCG GANAATCCCT ATCACTCAAG 540
AACAAGAACG GATCCCAGCC CTGANATCNA AGGAAATCTG CANCCTGCCA CACATGGCA 599

<210> 13

<211> 597
<212> DNA
<213> Homo sapiens

<400> 13
ATATCCGGAG TAGACGGAGC CGCAGTAGAC GGATCCGCGG CTGCACCAAA CACTGCCCCT 60
CGGAGCCTGG TAGTGGGCCA CAAGCCCCCA GTCCCAGAGG CGTGATTTTC TGGCATCCTT 120
AAATCTTGTG TCAAGGATTG GTTATAATAT AACCAGAAAC CATGACGGCG GCTGAGAACG 180
TATGCTACAC GTTAATTAAC GTGCCAATGG ATTCAGAACC ACCATCTGAA ATTAGCTTAA 240
AAAATGATCT AGAAAAAGGA GATGTAAAGT CAAAGACTGA AGCTTTGAAG AAAGTAATCA 300
TTATGATTCT GAATGGTGAA AAACTTCCTG GACTTCTGAT GACCATCATT CGTTTTGTGC 360
TACCTCTTCA GGATCACACT ATCAAGAAAT TACTTCTGGT ATTTTGGGAG ATTGTTCCTA 420
AAACAACTCC AGATGGGAGA CTTTTACATG AGATGATCCT TGTATGTGAT GCATACAGAA 480
AGGATCTTCA ACATCCTAAT GAATTTATTC NAAGGATCTA CTCTTCGTTT TCTTTGCAAA 540
TTGAAANAAA CANAATTGCT AAAACCTTTA ATGCCANCTA TNCCTGCATT TTTGGGA 597

<210> 14

<211> 634
<212> DNA
<213> Homo sapiens

<400> 14
AGACTCTCAC CGCAGCGGCC AGGAACGCCA GCCGTTCACG CGTTCGGTCC TCCTTGGCTG 60
ACTCACCGCC CTCGCCGCCG CACCATGGAC GCCCCCAGGC AGGTGGTCAA CTTTGGGCCT 120
GGTCCCGCCA AGCTGCCGCA CTCAGTGTTG TTAGAGATAC AAAAGGAATT ATTAGACTAC 180
AAAGGANTTG GCATTAGTGT TCTTGAAATG AGTCACAGGT CATCAGATTT TGCCAAGATT 240
ATTAACAATA CAGAGAATCT TGTGCGGGAA TTGCTAGCTG TTCCAGACAA CTATAAGGTG 300
ATTTTTCTGC AAGGAGGTGG GTGCGGCCAG TTCAGTGCTG TCCCCTTAAA CCTCATTGGC 360
TTGAAAGCAG GAANGTGTGC GGACTATGTG GTGACAGGAG CTTGGTCAGC TAAGGCCGCA 420
NAANAAGCCA AGAANTTTGG GACTATAAAT ATCGTTCACC CTAAACTTGG GAGTTATACA 480
AAAATTCCAG ATCCAAGCAC CTGGAACCTC AACCCAGATG CCTCCTACGT GTATTATTGC 540
GCNAATGAAA CNGTGCATGG TGTGGANTCT GACTTTATAC CCGATGTCNA GGGAACATAC 600
TGGTTTGTGA CATGTCCTCA AACTTCCCGT CCNA 634

<210> 15

<211> 757
<212> DNA
<213> Homo sapiens

<400> 15
AGTCTGCGGT GGGCTANCGG ACGGTCCGGC TTCCGGCGGC CGTTTCTGTC TCTTGCTGGC 60
TGTCTCGCTG AATCGCGGCC GCCTTCTCAT CGCTCCTGGA AGGTCCCGAG CGCGACACCA 120
TGTCGGAACC CGGGGGCGGC GGCGGCGAAG ACNGCTCGGC CGGATTGGAA GTGTCGGCCG 180
TGCANAATGT GGCGGACGTG TCGGTGCTGC ANAAGCACCT GCGCAAGCTG GTGCCGCTGC 240
TGCTGGAGGA CGGCGGCGAA GCGCCGGCCG CGCTGGAGGC GGCGCTGGAG GAGAAGAGCG 300
CCCTGGAGCA GATGCGCAAG TTCCTTTCGG ACCCGCACGT CCACACGGTG CTGGTGGAGC 360
GCTCCACGCT CAAAGTGGAC GTCGGTGATG AAGGAGAAGA AGAAAAAGAA TTCATTTCCT 420
ATAACATCAA CNTAGACATT CACTATGGGG TTAAATCCAA TAGCTTGGCA TTCATTAAAC 480
GTACTCCCGT GATTGATGCA GATAAACCCG TGTCTTCTCA NCTCCGGGTC CTTACACTCA 540

GTGAANACTC NCCCTACNAA AACTTTGCAT TCTTTCATTA ACAATGCAGT GGCTCCTTTT 600
TTTAANTCCT ACATTAAAAA ATCTGGCAAG GCAAACAGGG ATGGTGATAA AATGGCTCCT 660
TCCNTTGAAA AAAAAATTGC CGAACTCNAA ATNGGACTCC TTCCCTTGCA NCAAAATTTT 720
TGAAATTCCG GAAAATCANC CTGCCCAATT CCTCCCC 757

<210> 16

<211> 300
<212> DNA
<213> Homo sapiens

<400> 16
ATCATTTCCT TATTTATATT TCATGTTGGA ATGCTTAAAT CGATAACCTT TGTATTTTGA 60
AGTGCGCGAC ATGGAAGGTG ATCTGCAAGA GCTGCATCAG TCAAACACCG GGGGATAAAT 120
CTGGATTTGG GTTCCGGCGT CAAGGTGAAG ATAATACCTA AAGAGGAACA CTGTAAAATG 180
CCAGAAGCAG GTGAANAGCA ACCACAAGTT TAAATGAAGA CAAGCTGAAA CAACGCAAGC 240
TGGTTTTATA TTAGATATTT GACTTAAACT ATCTCAATAA AGTTTTGCAG CTTTCACCAC 300

<210> 17

<211> 313
<212> DNA
<213> Homo sapiens

<400> 17
AAAGATGGCG GCGGGGGAGG TAGGCAGAGC AGGACGCCGC TGCTGCCGCC GCCACCGCCG 60
CCTCCGCTCC AGTCGCCTCC GGTCCTTCAA ACTCACACCT CCCGGGAGGA GCTGTCCTGG 120
CGCCGGGTCC CGCGGGGAAA ATGGTGGAGC CAGGGCAAGA TTTACTGCTT GCTGCTTTGA 180
GTGAGAGTGG AATTAGTCCG AATGACTCTT TGATATTGAT GGTGGAGATG CANGGCTTGC 240
AACTCCAATG CCTACCCCGT CAGTTCAGCA NTCAGTGCCA CTTANTGCAT TANAACTANG 300
TTTGGAGACC GAA 313

<210> 18

<211> 667
<212> DNA

<213> Homo sapiens

<400> 18
ACTGCCGGGC TCGGCGTGAG TCGCTGCGGG GCTGACGGGG TGGCAGTGCG GCGGGTTACG 60
GCCTGGTCAG ACCATAATGA CTTCAGCAAA TAAAGCAATC GAATTACAAC TACAAGTGAA 120
ACAAAATGCA GAAGAATTAC AAGACTTTAT GCGGGATTTA GAAAACTGGG AAAAAGACAT 180
TAAACAAAAG GATATGGAAC TAAGAAGACA GAATGGTGTT CCTGAAGAGA ATTTACCTCC 240
TATTCGAAAT GGGAATTTTA GGAAAAAGAA GAAAGGCAAA GCTAAAGAGT CTTCCCCAAA 300
ACCANAGAGG AAAACACNAA AAACAGGATA AAATCTTATG ATTATGANGC ATGGGCAAAA 360
CTTGATGTGG ACCGTATCCT TGATGAGCTT GACAAAGACG ATAGTACCCA TGAGTCTCTG 420
TCTCAAGAAT CAGAGTCGGA AGAAGATGGG ATTCATGTTG ATTCNCNAAA GGCTCTTGTT 480
TTAAAAGAAA AGGGCNATAA ATACTTCCAC AAGGAAAATA TGATGAAGCA ATTGACTGCT 540
ACACNAAAGG CNTGGATGCC GATCCATATN ATCCCGTGTT GCCAACGAAC ANAACNTCCG 600
CATATTTTAG ACTGAAAAAA TTTGCTGTTG CTGAATCTGA TTGTTATTTAN CANTTGCCT 660
TGAAATA 667

## Description

### Technical field

The present invention belongs to the field of genetic engineering, and relates to a method for screening full-length cDNA clones.

### Background Art

Recently, genome projects targeting various animals, plants, and microorganisms have been in progress. Numerous genes have been isolated and their functions are under investigation. In order to efficiently analyze the functions of isolated genes, it is important to efficiently obtain cDNA clones capable of expressing complete proteins, that is, full-length cDNA clones.

The followings are known as methods for constructing a full length-enriched cDNA library: the oligo capping method in which an RNA linker is enzymatically bound to Cap of mRNA (Sugano & Maruyama, Proteins, Nucleic Acids and Enzymes, 38: 476-481, 1993, Suzuki & Sugano, Proteins, Nucleic Acids and Enzymes, 41: 603-607, 1996, M. Maruyama and S. Sugano, Gene, 138, 171-174, 1994); the modified oligo capping method developed by combining the oligo capping method with Okayama-Berg method (S. Kato et al., Gene, 150, 243-250, 1994, Kato & Sekine, Unexamined Published Japanese Patent Application (JP-A) NO. Hei 6-153953, published June 3, 1994); and the linker chemical-binding method in which a DNA linker is bound to Cap (N. Merenkova and D. M. Edwards, WO 96/34981 Nov. 7, 1996), the cap chemical modification method by biotin modification of Cap (P. Carninci et al., Genomics, 37, 327-336, 1996, P. Carninci et al., DNA Research, 4, 61-66, 1997). These are all methods to modify Cap of eukaryotic mRNA and to prepare a full length-enriched cDNA library. A known method for constructing a full length-enriched cDNA library by trapping Cap is the method using Cap-binding proteins derived from yeast or Hela cells for labeling a 5'-cap site (I. Edery et al., MCB, 15, 3363-3371, 1995). Also known is Cap Finder (Clontech) that is the Cap Switch oligonucleotide method in which the Cap Switch oligonucleotide is annealed by C-tailing the 5' end of a first strand cDNA.

A cDNA library constructed by these methods is rich in full-length cDNAs compared to that obtained by the conventional methods. However, incomplete-length clones are also contained to some extent. To efficiently analyze the functions of genes and to efficiently clone novel useful genes, development of methods for easily confirming whether each clone contained in a cDNA library is full-length or not has been desired.

### Disclosure of the Invention

An objective of the present invention is to provide a method for efficiently screening full-length cDNA clones, and a method for constructing a full length-enriched cDNA library.

The present inventors have studied to achieve the above objective and contemplated efficiently screening full-length cDNAs from a cDNA library by the presence or absence of a translation initiation codon as an index based on the fact that a cDNA deficient in a certain 5'-region is likely to lack a translation initiation codon, whereas a full-length cDNA contains an initiation codon. Especifically, the inventors assumed that a full-length cDNA could be efficiently screened from a cDNA library constructed by a method for preparing a full length-enriched cDNA library. Specifically, the inventors thought that full-length cDNA clones could be efficiently isolated by constructing a cDNA library by a method for preparing a full length-enriched cDNA library, determining several hundreds of base pairs of a DNA nucleotide sequence from the 5' end, and analyzing the presence or absence of an initiation codon in this region to screen the clones containing initiation codons.

However, few programs for predicting an initiation site of cDNA have been developed (e.g., "A. G. Pedersen, Proceedings of fifth international conference on intelligent systems for molecular biology, p226-233, 1997, held in Halkidiki, Greece, June 21-26, 1997). Though some programs for exons prediction have been developed ("Gene Finder". V. V. Solovyev et al., Nucleic Acids Res., 22, 5156-5163, 1994, "Grail" Y. Xu et al., Genet-Eng-N-Y., 16, 241-253, 1994), an initiation site cannot be accurately determined relying solely on these programs.

The present inventors have developed a program for cDNA initiation codon prediction by themselves and determined nucleotide sequences of the 5'-region of clones contained in a cDNA library constructed by a method for preparing a full length-enriched cDNA library to examine whether an initiation codon exists in this 5'-region using this software program.

More specifically, a full length-enriched cDNA library was constructed by the oligo capping method and nucleotide sequences of the 5'-regions of some clones contained in the cDNA library were determined. Based on the determined sequences, the clones were divided into known and novel ones through a database search. The presence or absence of an initiation codon and its location in the determined nucleotide sequences of the 5'-regions were judged using the initiation codon prediction program. For the known clones, whether the location of the initiation codon recognized by the initiation codon prediction program coincides with that of the initiation codon in databases is examined. Indeed, the presence or absence and location of the initiation codon in the known clones predicted by the program coincided with the information in the databases.

Thus, the software program developed by the present inventors can accurately recognize the presence or absence of an initiation codon and its location, and full-length cDNA clones can be efficiently screened by selecting the clones that are recognized to contain an initiation codon by the program from the cDNA library. Moreover, a cDNA library extremely rich in full-length cDNAs can be constructed by combining the screened clones.

The present invention relates to a method for screening full-length cDNA clones from a cDNA library and a method for constructing a full-length cDNA library by combining cDNA clones screened by the screening method. More specifically, it relates to:
(1) A method for isolating a full-length cDNA clone, the method comprising:
   (a) determining a nucleotide sequence from the 5'-region of a cDNA clone contained in a cDNA library,
   (b) determining the presence or absence of an initiation codon in the nucleotide sequence determined in (a) using an initiation codon prediction program, and
   (c) selecting clones recognized as containing the initiation codon in (b);
(2) The method of (1), wherein the cDNA library is constructed by a method for preparing a full length-enriched cDNA library;
(3) The method of (1), wherein a cDNA library is constructed by a method comprising a step of modifying Cap of mRNA;
(4) A method for constructing a full length cDNA library, the method comprising:
   (a) determining a nucleotide sequence from the 5'-region of a cDNA clone contained in a cDNA library,
   (b) determining the presence or absence of an initiation codon in the nucleotide sequence determined in (a) using an initiation codon prediction program,
   (c) selecting clones recognized as containing the initiation codon in (b), and
   (d) combining the clones selected in (c);
(5) The method of (4), wherein the cDNA library is prepared by a method for constructing a full length-enriched cDNA library;
(6) The method of (4), wherein the cDNA library is constructed by a method comprising a step of modifying Cap of mRNA; and
(7) A cDNA library obtainable by the method of (4).

The present invention is based on the inventors' findings that full-length cDNA clones can be efficiently isolated by analyzing nucleotide sequences of the 5-region of cDNAs in a cDNA library, specifically a full length-enriched cDNA library, by using a software program for accurately predicting a translation initiation codon, and a full length-enriched cDNA library can be constructed by combining the isolated cDNA clones. The method for screening full-length cDNA clones by the present invention comprises (a) determining a nucleotide sequence from the 5'-region of a cDNA clone contained in a cDNA library, (b) determining the presence or absence of an initiation codon in the determined nucleotide sequence using an initiation codon prediction program, and (c) selecting clones recognized as containing the initiation codon. The method for constructing a full-length cDNA library of the present invention comprises, in addition to above steps (a) to (c), step (d) of combining the screened clones.

In the method of the present invention, a "cDNA clone" whose nucleotide sequence of the 5'-region is to be determined is not particularly limited. Full-length cDNAs cannot be efficiently isolated from clones derived from a library not rich in full-length cDNAs, compared with clones derived from a full length-enriched cDNA library. Therefore, a cDNA clone is preferably derived from a library constructed by the above-described methods for preparing a full length-enriched cDNA library, including, for example, the oligo capping method in which an RNA linker is enzymatically bound to Cap of mRNA (Sugano & Maruyama, Proteins, Nucleic Acids and Enzymes, 38: 476-481, 1993, Suzuki & Sugano, Proteins, Nucleic Acids and Enzymes, 41: 603-607, 1996, M. Maruyama and S. Sugano, Gene, 138, 171-174, 1994), the modified oligo capping method developed by combining the oligo capping method with Okayama-Berg method (S. Kato et at, Gene, 150, 243-250, 1994, Kato & Sekine, JP-A-Hei 6-153953, June 3, 1994), the linker chemical-binding method in which a DNA linker is chemically bound to Cap (N. Merenkova and D. M. Edwards, WO 96/34981 Nov. 7, 1996), the Cap chemical modification method in which Cap is modified with biotin (P. Carninci et al., Genomics, 37, 327-336, 1996, P. Carninci et al., DNA Research, 4, 61-66, 1997), the method using Cap binding proteins drived from yeast or Hela cells (I. Edery et al, MCB, 15, 3363-3371, 1995), or a library prepared by Cap Finder using Cap Switch oligonucleotide method.

A cDNA clone can be isolated from a cDNA library by standard methods described in, for example, J. Sambrook, E. F. Fritsch & T. Maniatis, Molecular Cloning, Second Edition, Cold Spring Harbor Laboratory Press, 1989.

A nucleotide sequence can be determined from the 5'-region of a clone by, for example, standard methods using DNA sequencing reagents and a DNA sequencer available from Applied Biosystems, etc. A whole nucleotide sequence of the clone dose not have to be determined, and determining about 1,000 nucleotides from the 5' end is sufficient. The high accuracy can be expected by determining about 500 nucleotides, even about 300 nucleotides.

An "initiation codon prediction program" used for analyzing a nucleotide sequence from the 5'-region of a clone is preferably the program developed by the present inventors as described in Example 1 below. The presence or absence of an initiation codon in a determined sequence is judged by a score deduced from the results of analysis with the program. A cDNA clone with a high score, recognized as containing an initiation codon in the determined sequence, is usually comprised of a full-length cDNA, while one with a low score, recognized as not containing an initiation codon in the determined sequence, contains an incomplete-length cDNA. Thus, a full-length cDNA can be efficiently isolated by screening a cDNA from a cDNA library, judged as containing an initiation codon in the nucleotide sequence. Indeed, in one embodiment of the analysis with the program described in Example 1 below where a cDNA library with the full-length cDNA content of 51% was used to screen clones (the highest score was 0.94), the content of full-length clones among the screened clones was 71% when clones showing a score of 0.5 or higher were selected, 77% with a score of 0.70 or higher, 81% with a score of 0.80 or higher, and 85% with a score of 0.90 or higher. Therefore, full-length cDNA clones can be screened with a high accuracy by selecting clones with high scores using the program described in Example 1.

Moreover, a cDNA library re-constructed by combining clones selected by the method for screening full-length cDNA clones of the present invention is extremely rich in full-length cDNAs compared with the parent cDNA library used for screening clones. By expressing whole cDNAs capable of expressing proteins in the thus-obtained library, a system for efficiently analyzing gene functions containing a mixture of expressed proteins can be obtained. This system enables efficiently cloning useful genes.

### Best Mode for Carrying out the Invention

The present invention is illustrated in detail below with reference to the following examples, but is not to be construed as being limited thereto.

### Example 1. Preparation of a program for predicting a translation initiation codon of cDNA

The translation initiation codon prediction program of the present invention recognizes a putative authentic initiation codon among all ATGs contained in a given cDNA sequence fragment. The program predicts based on A) information on similarity of given regions (several tens to several hundreds base pairs) at both sides of a putative ATG to translational regions and B) information on similarity of regions near a putative ATG to those near an authentic initiation codon. Characteristics of sequences in a translational region and regions near an initiation codon are extracted beforehand by from information of numerous sequences whose translational and non-translational regions have been identified. The program predicts an initiation codon based on the information about the above characteristics.

The linear discriminant analysis used in Gene Finder, a program for genomic exon prediction (Solovyev V. V., Salamov A. A., Lawrence C. B. Predicting internal exons by oligonucleotide composition and discriminant analysis of spliceable open reading frames. Nucleic. Acids Res, 1994, 22: 5156-63), was applied to optimize prediction. In the linear discriminant analysis, information on some characteristics derived from data is digitizied, weighted, and then culculated a score. Here, a score is converted into a probability of similarity to an initiation codon (the probability is a rate of correct answers obtained from data of sequences whose initiation codon has been identified). Specifically, a probability of similarity to an initiation codon of each ATG contained in a given cDNA sequence is output. Recognition as an initiation codon is determined whether a probability of similarity to an initiation codon is above a given threshold value or not. A threshold value is established depending on the plan of the following analyses, that is, depending on the extent of noises acceptable for the following analysis. For example, when 40% of noise is acceptable, a threshold value of 0.6 can be used. A parameter of weight is determined so as to maximize the prediction system using data of sequences whose initiation codon has been identified as a training datum. The above information of A) and B) were each embodied into the following three information and used as information about characteristics.

### A) information on similarity of given regions (several tens to several hundreds base pairs) at both sides of a putative ATG to translational regions

1: a frequency of six nucleotide base letters contained in a sequence from ATG to a stop codon (within 300 bp downstream of ATG at longest)
2: discrepancy of the information on a frequency of six nucleotide base letters contained in 50 nucleotide bases upstream and downstream of ATG
3: an index of similarity to a signal peptide [a hydrophobicity index of the most hydrophobic eight amino acids letters among 30 amino acids (90 nucleotide bases) downstream of ATG]

### B) information on similarity of regions near a putative ATG to those near an authentic initiation codon

1: information on a weighted matrix as using three nucleotide base letters in the region from 14 nucleotide bases upstream of ATG to 5 nucleotide bases downstream of ATG as a unit
2) the presence or absence of other ATGs upstream of ATG in a same frame (the presence is 1 and the absence is 0)
3: a frequency of cytosine contained in the region from 36 bases upstream of ATG to 7 bases downstream of ATG.

### Example 2: Preparation of cDNA by the oligo capping method and analysis thereof by the program for initiation codon prediction

A cDNA library was prepared by the oligo capping method and the plasmid DNA was extracted from each clone by the standard method. Specifically, mRNA was extracted from human placenta and human cultured cells (Tetratocarcinoma NT-2 and neuroblatoma SK-N-MC) by the method described in the reference (J. Sambrook, E. F., Fritsch & T. Maniatis, Molecular Cloning, Second Edition, Cold Spring Harbor Laboratory Press, 1989). An oligo cap linker (SEQ ID NO. 1) with an oligo dT adaptor primer (SEQ ID NO. 2) in the case of Tables 1 & 2, or with a random adaptor primer (SEQ ID NO. 3) in the case of Tables 3 & 4 were subjected to BAP treatment, TAP treatment, RNA ligation, synthesis of a first strand cDNA, and removal of RNA according to the methods described in the references (Suzuki & Sugano, Proteins, Nucleic Acids, and Enzymes, 41, 603-607, 1996, p606, Y. Suzuki et al., Gene, 200, 149-156, 1997). The first strand cDNA was then converted into the double-stranded DNA by PCR, digested with *SFi*I, and cloned into vectors, such as pME18SCG, pMFL etc. digested with *Dra*III in the determined direction (Sugano & Maruyama, Proteins, Nucleic Acids, and Enzymes, 38, 472-481, 1993, p480). The obtained DNA was subjected to the sequencing reaction using a DNA sequencing reagent (DyeTerminatoir Cycle Sequencing FS Ready Reaction Kit, PE Applied Biosystems) following the manual and sequenced with a DNA sequencer (ABIPRISM 377, PE Applied Biosystems). The DNA sequence of the 5'-region of each clone was analyzed once.

The presence or absence of an initiation codon in the DNA sequence of each clone was analyzed using the developed program for cDNA initiation codon prediction (ATGpr). In this analyzing program, the higher the score is, the higher the probability of being an initiation codon is. The maximum score is 0.94.

### (1) Analysis of translation initiation codons in the clones whose open reading frames are known in database among cDNA prepared by the oligo capping method

Among the results for all analyzed clones, the result for the clones that are known to contain the initiation codon in the determined sequences in databases (F-NT2RP1000020, F-NT2RP1000025, F-NT2RP1000039, and F-NT2RP1000046) are shown in Table 1. F-NT2RP1000020 (880 bp) has 96% identity at nucleotide positions 88 to 690 to "human neuron-specific gamma-2 enolase" (GenBank accession No. M22349); F-NT2RP1000025 (645 bp), 97% homology at positions 29 to 641 to "human alpha-tubulin mRNA" (GenBank accession No. K00558); F-NT2RP1000039 (820 bp), 96% identity at positions 12 to 820 to "human mRNA for elongation factor 1 alpha subunit (EF-1 alpha) (GenBank accession No. X03558); and F-NT2Rp1000046 (788 bp), 97% identity at positions 3-788 to "human M2-type pyruvate kinase mRNA" (GenBank accession No. M23725). The sequences of the 5'-region in these clones are shown in SEQ ID Nos: 4, 5, 6, and 7.

**Table 1**

| | F-NT2RP1000020 | | F-NT2RP1000025 | | F-NT2RP1000039 | | F-NT2RP1000046 | |
|---|---|---|---|---|---|---|---|---|
| ATG No. | Location of ATG | ATGpr Score | Location of ATG | ATGpr Score | Location of ATG | ATGpr Score | Location of ATG | ATGpr Score |
| 1 | 1 | 0.05 | 96 | 〈0.94〉 | 65 | 〈0.90〉 | 111 | 〈0.94〉 |
| 2 | 162 | 〈0.84〉 | 148 | 0.13 | 154 | 0.05 | 174 | 0.82 |
| 3 | 292 | 0.05 | 193 | 0.05 | 209 | 0.11 | 198 | 0.19 |
| 4 | 313 | 0.05 | 201 | 0.09 | 231 | 0.05 | 300 | 0.16 |
| 5 | 441 | 0.05 | 232 | 0.05 | 321 | 0.05 | 315 | 0.11 |
| Note 1: 〈〉 means translation initiation codon Note 2: Location of ATG means the nucleotide base position of ATG in the 5'-region of a DNA sequence. ATG No. means the number of ATG from the 5'-region of a DNA sequence. | | | | | | | | |

As show in Table 1, among the cDNA prepared by the oligo capping method, the full-length clones whose open reading frames are known in databases, containing initiation codons were accurately recognized by the initiation codon prediction program (ATGpr) (coincident with the initiation codons in databases).

### (2) Analysis of initiation codons in the clones whose open reading frames are known in database among cDNA prepared by the oligo capping method

Among the results for the clones analyzed, the results for the clones whose initiation codon is known to absent in the determined sequence in databases (F-NT2RP1000013, F-NT2RP1000054, and F-NT2RP1000122) are shown in Table 2. F-NT2RP1000013 (608 bp) has 97% identity at positions 1 to 606 to "human nuclear matrix protein 55 (nmt55) mRNA" (GenBank accession No.U89867); F-NT2RP1000054 (869 bp), 96% identity at positions 1 to 869 to "human signal recognition particle (SRP54) mRNA" (GenBank accession No. U51920); and F-NT2RP1000122 (813 bp), 98% identity at positions 1 to 813 to *"H. sapiens* mRNA for 2-5A binding protein" (GenBank accession No. X76388). The sequences of the 5' region of these clones are shown in SEQ ID Nos: 8, 9, and 10.

**Table 2**

| | F-NT2RP1000013 | | FNT2RP1000054 | | F-NT2RP1000122 | |
|---|---|---|---|---|---|---|
| ATG No. | Location of ATG | ATGpr Score | Location of ATG | ATGpr Score | Location of ATG | ATGpr Score |
| 1 | 21 | 0.05 | 31 | 0.12 | 23 | 0.07 |
| 2 | 27 | 0.05 | 60 | 0.20 | 100 | 0.05 |
| 3 | 32 | 0.32 | 87 | 0.05 | 166 | 0.05 |
| 4 | 56 | 0.11 | 97 | 0.05 | 235 | 0.06 |
| 5 | 119 | 0.10 | 146 | 0.05 | 316 | 0.05 |
| 6 | 125 | 0.08 | 172 | 0.05 | 346 | 0.05 |
| 7 | 141 | 0.05 | 180 | 0.11 | 406 | 0.05 |
| 8 | 155 | 0.06 | 218 | 0.07 | 431 | 0.05 |
| 9 | 161 | 0.06 | 272 | 0.05 | 469 | 0.06 |
| 10 | 176 | 0.08 | 319 | 0.07 | 546 | 0.12 |
| 11 | 203 | 0.07 | 346 | 0.05 | 553 | 0.05 |
| 12 | 290 | 0.20 | 363 | 0.07 | 574 | 0.05 |
| 13 | 311 | 0.16 | 409 | 0.05 | | |
| 14 | 314 | 0.12 | 480 | 0.07 | | |

As shown in Table 2, among cDNA prepared by oligo capping method, the initiation codon prediction program (ATGpr) did not recognize by mistake the initiation codons in incomplete-length cDNAs whose open reading frames are known in databases and which do not contain any initiation codons.

### (3) Analysis of initiation codons in novel clones among the cDNA prepared by the oligo capping method

Among the results for analyzed clones, the results for novel clones that were predicted to contain initiation codons (F-ZRV6C1000408, F-ZRV6C1000454, F-ZRV6C1000466, F-ZRV6C1000615, and F-ZRV6C1000670) are shown in Table 3. The sequences of the 5' region of these clones are shown in SEQ ID Nos: 11, 12, 13, 14, 15.

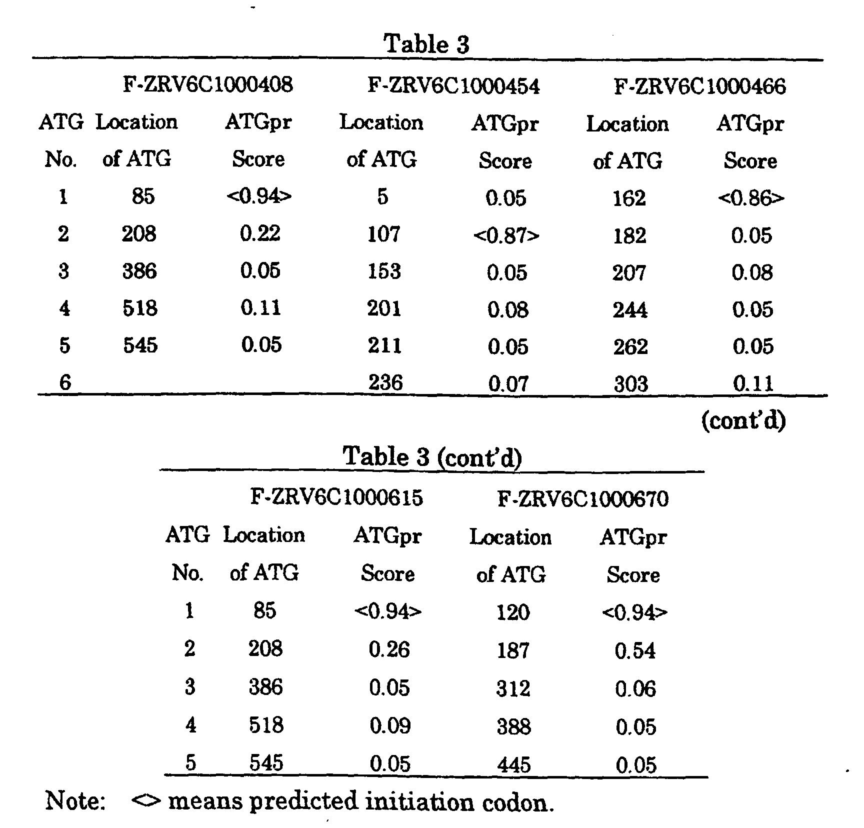

As shown in Table 3, the predicted initiation codons in F-ZRV6C1000408, F-ZRV6C1000454, F-ZRV6C1000466, F-ZRV6C1000615, and F-ZRV6C1000670 are "ATG" starting with "A" at positions 85, 107, 162, 85, and 120, respectively. Therefore, these clones were judged as full-length cDNA clones.

In addition, among the results for analyzed clones the results for novel clones predicted as not containing initiation codons (F-ZRV6C1001410, F-ZRV6C1001197, and F-ZRV6C1001472) are shown in Table 4. The sequences of the 5' region of these clones are shown in SEQ ID Nos: 16, 17 and 18.

**Table 4**

| | F-ZRV6C1001410 | | F-ZRV6C1001197 | | F-ZRV6C1001472 | |
|---|---|---|---|---|---|---|
| ATG No. | Location of ATG | ATGpr Score | Location of ATG | ATGpr Score | Location of ATG | ATGpr Score |
| 1 | 23 | 0.05 | 5 | 0.24 | 77 | 0.25 |
| 2 | 31 | 0.07 | 141 | 0.25 | 126 | 0.05 |
| 3 | 71 | 0.06 | 202 | 0.05 | 149 | 0.05 |
| 4 | 178 | 0.05 | 219 | 0.05 | 194 | 0.05 |
| 5 | 214 | 0.05 | 228 | 0.05 | 213 | 0.22 |
| 6 | | | | | 249 | 0.05 |
| 7 | | | | | 338 | 0.09 |
| 8 | | | | | 344 | 0.05 |
| 9 | | | | | 351 | 0.05 |
| 10 | | | | | 365 | 0.05 |

As shown in Table 4, F-ZRV6C1001410, F-ZRV6C1001197, and F-ZRV6C1001472 were recognized as not containing initiation codons. These clones were thus judged as incomplete-length clones.

### Industrial Applicability

The present invention provides a method for efficiently selecting full-length cDNAs. Clones selected by the method of the present invention can express complete proteins. Therefore, the present invention enables efficiently analyzing the functions of isolated genes.

## Claims

1. A method for isolating a full-length cDNA clone, the method comprising:
(a) determining a nucleotide sequence from the 5'-region of a cDNA clone contained in a cDNA library;
(b) determining the presence or absence of an initiation codon in the nucleotide sequence determined in (a) using an initiation codon prediction program; and
(c) selecting clones recognized as containing the initiation codon in (b).

2. The method of claim 1, wherein the cDNA library is constructed by a method for preparing a full length-enriched cDNA library.

3. The method of claim 1, wherein a cDNA library is constructed by a method comprising a step of modifying Cap of mRNA.

4. A method for constructing a full length cDNA library, the method comprising:
(a) determining a nucleotide sequence from the 5'-region of a cDNA clone contained in a cDNA library;
(b) determining the presence or absence of an initiation codon in the nucleotide sequence determined in (a) using an initiation codon prediction program;
(c) selecting clones recognized as containing the initiation codon in (b); and
(d) combining the clones selected in (c).

5. The method of claim 4, wherein the cDNA library is prepared by a method for constructing a full length-enriched cDNA library.

6. The method of claim 4, wherein the cDNA library is constructed by a method comprising a step of modifying Cap of mRNA.

7. A cDNA library obtainable by the method of claim 4.
